# EUROPEAN PATENT APPLICATION

(11) **EP 4 104 753 A1**
(43) Date of publication of application: **21.12.2022**
(21) Application number: 22179178.3
(22) Date of filing: 15.06.2022
(51) Int. Cl.: A61B 5/00

(54) **DEVICE FOR MEASURING SENSITIVITY IN A SUBJECT**

(30) Priority: 18.06.2021 CA 3122747
(71) Applicant: 5010889 Ontario Inc., Sudbury P3A 2A4 (CA)
(72) Inventor: ROSSI, Shawn, Sudbury, P3A 2A4 (CA)
(74) Representative: Schmidt, Christian

(57) **Abstract**

Disclosed are devices and systems designed to assist an examiner in assessing the level of pain sensitivity in a patient, along with methods of using of the devices and/or systems. The device contains a tip for applying pressure to a point of the patient's body; a pressure sensor used to measure the pressure; a displacement sensor used to measure the depth of probing; and an optional third sensor used to measure temperature. The device can therefore simultaneously measure the pressure applied by the probe and the distance travelled by the tip of said probe (i.e. the displacement achieved). The device can be included in a system, which receives the data from the aforementioned sensors, and the patient's perceived pain value on a scale of 1-10. These values can be analyzed over time (i.e. over multiple applications of the device) to permit the examiner to use the data obtained to assess potential changes in the level of pain or discomfort and/or amount of healing of the patient.

## Description

### FIELD OF THE INVENTION

The invention generally relates to pain-measurement devices. More specifically, the invention relates to a pain-measurement device comprising sensors for detecting at least the pressure administered and depth reached by an associated probe, and methods of using said device.

### BACKGROUND OF THE INVENTION

Dolorimeters, instruments used to measure pain threshold and pain tolerance and/or sensitivity in a subject, are well known in the art. Dolorimeters typically apply pressure, heat, or electrical stimulation to an area of the body, or move a joint or other body part, and determine what level of heat, pressure, electric current or amount of movement produces a sensation of pain. The pressure may be applied in a variety of manners, such as using a blunt object, by pressing a sharp instrument against the body, or by locally increasing the air pressure on a body area. Algometers are devices that can be used to identify the pressure and/or force eliciting a pressure-pain threshold.

It has been noted in pressure-pain threshold studies that the rate at which manual force is applied should be consistent to provide the greatest reliability.

Current methods used in the art to measure healing, assess pain levels, and access injuries require an examiner or practitioner to use manual palpation into an area of a patient, and subsequently to ask the patient for their resulting perceived pain level on a scale from 1 to 10. This allows for a wide range of variation of results from one practitioner to another and a lack of standardization makes it difficult to effectively evaluate whether improvement has occurred from one assessment to the next.

While there are devices known in the art (algometers, dolorimeters etc.) that can measure the pressure applied to the body of a subject, there is a lack of such devices that measure both the pressure applied and the depth/displacement of the probe. Therefore, there is a need for improved devices that can simultaneously measure applied pressure, depth/displacement and/or temperature, so that standardization of these measurements can be created in order to improve the evaluation of pain and healing of a subject.

### SUMMARY OF THE INVENTION

It is an object of the present invention to provide a hand-held algometer device that includes sensors for the measurement of the force/pressure, depth and, optionally temperature of a component probe, which allows practitioners to give an empirical value to the amount of pressure or force applied and displacement or depth achieved by the device, which permits a degree of standardization resulting in an improved evaluation of pain levels and healing of a patient or subject.

According to an aspect of the present invention there is provided a hand-held device for taking measurements related to sensitivity in a subject. The hand-held device comprising: a housing having a subject engagement end for positioning the device against a target area of the subject's body; a probe encompassed by the housing and moveable from a first position where the probe is contained within the housing to a second position where one end of the probe extends beyond the subject engagement end of the housing; an activator positioned on an exterior surface of the housing and connected to the probe for moving the probe between the first and second positions; a displacement sensor connected to the probe for measuring the distance the probe travels between the first and second positions; and a pressure sensor connected to the probe for measuring the force applied to move the probe between the first and second positions.

In one embodiment, the hand-held device further comprising a microprocessor connected to the displacement and pressure sensors for receiving measurements from the displacement and pressure sensors; and an output for relaying information from the microprocessor to a user.

In another embodiment, the displacement sensor comprises a rod having distance intervals marked thereon to provide a visual indication of the distance the probe travels between the first and second positions.

In a further embodiment, the hand-held device further comprising a temperature sensor connected to the one end of the probe for measuring the temperature of the target area of the subject's body.

In yet a further embodiment, the temperature sensor is connected to the microprocessor.

In a still further embodiment, the output is a transmitter or a display positioned on the exterior of the housing.

In another embodiment, the transmitter is a WiFi or Bluetooth^{®}.

In a further embodiment, the output is a wire connected to computer or tablet.

In a still further embodiment, the wire is removably connected to the output and/or computer or tablet.

In yet another embodiment, a portion of the exterior surface of the housing forms a grip for the user's hand.

In a further embodiment, the activator is positioned on or near the end of the housing opposite the subject engagement end.

In a still further embodiment, the subject engagement end of the housing comprises a plate to surround the target area of the subject's body.

In another embodiment, the displacement sensor and pressure sensor are piezoelectric sensors.

According to another aspect of the invention, there is provided a system for measuring and tracking sensitivity in a subject. The system comprising: the hand-held device described above; a microprocessor connected to the hand-held device; an input device connected to the microprocessor for inputting data related to the subject's sensitivity to the probe being pressed against the target area; and a display connected to the microprocessor for displaying a set of values.

In one embodiment, the microprocessor is wireless connected to the hand-held device.

In another embodiment, the microprocessor is contained within a computer, tablet or cellphone.

In a further embodiment, the set of values relates to one or more of the force measured by the pressure sensor, the distance travelled by the probe, the temperature of the target area and the data related to the subject's sensitivity to the probe being pressed against the target area.

In a still further embodiment, the microprocessor comprises computer-readable medium for storing the set of values.

According to another aspect of the present invention, there is provided use of the device described above for taking measurements related to sensitivity in a subject.

According to yet another aspect of the present invention, there is provided use of the system described above for taking measurements related to sensitivity in a subject.

According to a further aspect of the present invention, there is provided a method of taking measurements related to sensitivity in a subject comprising positioning the device described above against a target area on a subject's body; and pressing the activator to move the probe from the first position to the second position.

### BRIEF DESCRIPTION OF THE DRAWINGS

The embodiments of the present disclosure will be described with reference to the following drawings in which:
FIG. 1 is an upper perspective, and partially cutout, view of an embodiment of the device in an unengaged position;
FIG. 2 is an upper perspective, and partially cutout, view of an embodiment of the device in the engaged position;
FIG. 3 is isolated view of an embodiment of the device; and
FIG. 4 is a graphical representation of an embodiment of a system in operation to take measurements related to sensitivity in a subject.

### DETAILED DESCRIPTION OF THE INVENTION

The following description is of a preferred embodiment by way of example only and without limitation to the combination of features necessary for carrying the invention into effect.

The present invention relates to a device that takes measurements related to sensitivity in a subject. These measurements include the depth in which a probe extends from the device into a surface of a subject's body and the pressure exerted by the probe against the surface of a subject's body. Optionally, a subject's sensitivity to temperature can be measured by placing a thermoelectric element in the tip, or end of the probe that is pressed against the surface of a subject's body. The use of depth, pressure, and optionally heat, can provide a reliable measurement of a subject's sensitivity to these parameters. These measurements can then be used to determine a subject's pain tolerance.

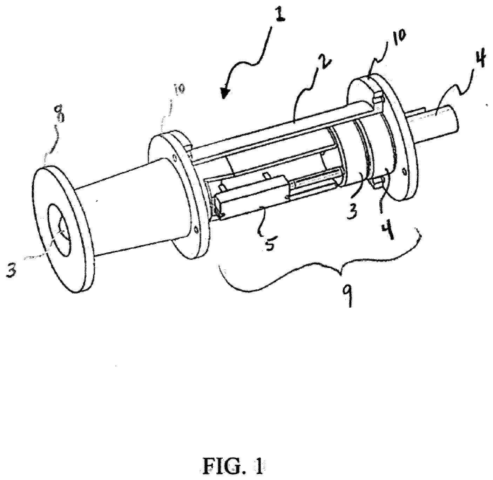

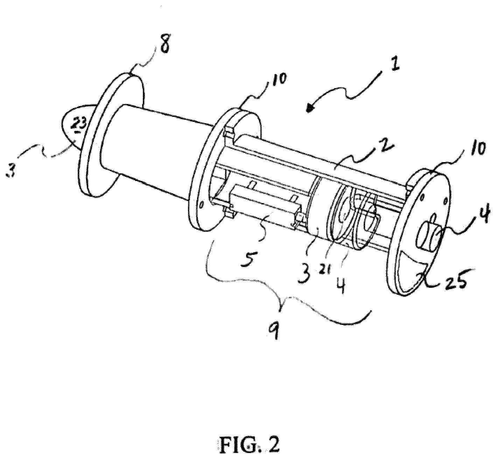

As shown in FIG. 1, the device (1) described herein has a housing (2), a probe (3) encompassed by the housing (2), an activator (4) positioned on an exterior surface of the housing (2) and connected to the probe (3), a displacement sensor (5) connected to the probe (3) and a pressure sensor (6) connected to the probe (3). In particular, the device (1) is a hand-held device having a housing (2) having a subject engagement end (7) for positioning the device (1) against a target area of the subject's body; a probe (3) encompassed by the housing (2) and moveable from a first position where the probe (3) is contained within the housing (2) (see FIG. 1) to a second position where one end of the probe extends beyond the subject engagement end (7) of the housing (2)(see FIG. 2); an activator (4) positioned on an exterior surface of the housing (2) and connected to the probe (3) for moving the probe (3) between the first and second positions; a displacement sensor (5) connected to the probe (3) for measuring the distance the probe (3) travels between the first and second positions; and a pressure sensor (6) connected to the probe (3) for measuring the force applied to move the probe (3) between the first and second positions.

The housing (2) is shaped to allow the user to comfortably grasp the device. In one embodiment, the housing (2) is cylindrical in shape with one end configured to be positioned against a target area of the subject's body. In some embodiments, the end of the housing (2) that engages the target area of the subject's body is provided with a plate (8) that surrounds the target area. The use of a plate (8) allows the device (1) to be pressed substantially flat against the subject's body, thus allowing the probe (3) to move essentially perpendicular to the target area. Without the use of a plate (3), the user of the device (1) may advance the probe (3) at an angle to the target area causing user variability in the readings. However, in some cases, a device (1) without a plate (8) may be required to obtain measurements from a target area that is located on the subject's body either at a spot that is too small to accommodate a device (1) having a plate (8) or if the target area is on a part of the body that is not essentially flat, such as the shoulder. To accommodate both situations, the plate (8) may be detachable. Suitable methods for detaching the plate (8) will be known to a person skilled in the art, and include, but are not limited to, screw-on and compression fittings.

As shown in the Figures, the housing (2) may be sectioned to include a grip portion (9) that is defined by two stops (10) that limits the user's hand from being displaced from the device (1) when the probe (3) applies pressure to the target area. As shown, the stops (10) can be provided as flanges provided at each end of the grip portion (10). In some cases, the grip portion (10) will be defined on the housing (2) by the exterior surface of the housing (2) having a different texture, typically having a higher degree of resistance, than the rest of the housing.

The housing (2) encompasses a probe (3) which is moveable from a first position, where the probe is contained within the housing (see FIG. 1), to a second position, where one end of the probe extends beyond the subject engagement end of the housing (see FIG. 2). In one embodiment, the probe (3) is a cylinder that is surrounded by the housing (2) and that moves independently from the housing (2). The end of the probe (3) that is pressed against the target area of the subject's body can be provided in many forms. However, to avoid the probe (3) from piercing the subject's body, it is preferred that the end of the probe (3) that is pressed against the target area is provided in a size and shape that is not considered sharp, such as a rounded shape.

In one embodiment, the end of the probe (3) that is pressed against the target area of the subject's body can include a thermoelectric element (not shown) capable of producing both heat and cold. The presence of a thermoelectric element in the tip of the probe (3) allows for the subject's sensitivity to either heat or cold to be tested.

In yet a further embodiment, a temperature sensor (not shown), such as pyrometric sensor, can be included in the end of the probe (3) that is pressed against the target area of the subject's body in order to measure the temperature of the target area on the subject's body. This data can be fed into the system described below as a further variable to be used to determine a subject's sensitivity to pain or other elements, such as heat and cold. This can be particularly advantageous since the inflammatory response to an injury often results in the skin surface temperature surrounding the injury to be increased. As such, sensitivity measurements can be affected by the skin surface temperature of the target area.

In operation, an activator (4) positioned on the exterior surface of the housing (2) causes the movement of the probe (3) from the first position, where the probe (3) is contained within the housing (2), to a second position, where the end of the probe (3) that is pressed against the target area of the subject's body extends beyond the subject engagement end (7) of the housing (2). In one embodiment, the activator (4) is positioned on the end of the housing (2) opposite the subject engagement end (7) and is pressed towards the housing (2) to move the probe (3) from the first position to the second position.

As shown in FIG. 2, the pressure or force sensor (21) can be sandwiched between the activator (4) and the end of the probe (3) opposite the end that is pressed against the target area of the subject's body. This arrangement allows for the pressure applied by the activator (4) while moving the probe (3) from the first position to the second position to be measured.

Pressure sensors (21) used in the device (1) should be capable of measuring the amount of pressure that is created between the probe (3) and the activator (4). The resulting pressure will be an indication of resistance provided by the subject's body at the target area to movement of the probe (3). Suitable pressure sensors (21) for use in the device (1) can include, but are not limited to, piezoelectric sensors, load cells, resistive force sensors, capacitive force sensors and piezo crystal type sensors.

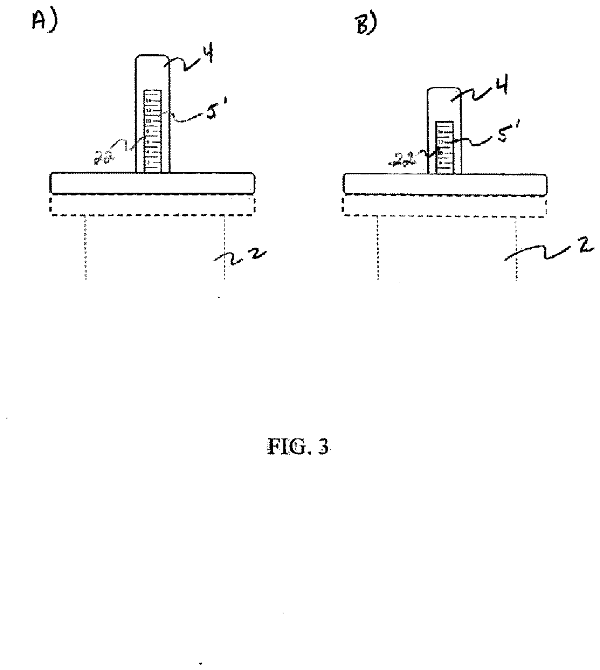

In addition to the pressure sensor (21), the device described herein also contains a displacement sensor (5) to measure the distance the probe (3) travels while moving from the first position to the second position. In one embodiment, the displacement sensor (5) can be connected to the probe (3) whereas in other embodiments, such as that shown in FIG. 3, the displacement sensor (5') can be connected to the activator (4). As shown in FIGs. 1 and 2, the displacement sensor (5) can be connected to the probe (3) so that when the probe (3) moves from the first position to the second position, the displacement sensor (5, 5') measures the movement of the probe (3) with respect to the rest of the housing (2). In one embodiment, the displacement sensor (5) can include a variable resistor that changes resistance based on the position of the actuator. The variable resistor containing a spring inside that moves the wiper on a potentiometer. In addition, other displacement sensors, such as, but not limited to, an infra-red transmitter and receiver pair, a variable distance capacitive plate system, ultrasonic distance sensor, optical or video, time of flight, laser, inductive proximity, magnetic proximity, motor or shaft encoder systems can be used to determine the distance travelled by the probe between the first and second positions.

In other embodiments, the displacement sensor (5') can be attached to the activator (4), since the distance travelled by the activator (4) is directly proportional to the distance travelled by the probe (3) when moving from the first position to the second position. In this case, the displacement sensor (5') can be in the form of a rod that is connected to the activator (4). The rod can be attached to the activator (4) at a point inside the housing (2) and dimensioned so that the rod passes through the housing (2) and terminates at a position outside the housing (2) (see FIG. 3). Markings (22) relating to distance intervals can be provided on the rod so that when the probe/activator moves from the first position to the second position, a set number of markings (22) disappears into the housing (2) corresponding to the distance travelled by the probe (3) from the first (see FIG. 3A) to the second position (FIG. 3B). Alternatively, the markings (22) could be provided on the activator (4) itself.

In one embodiment, the tip (23), or end of the probe (3) that is pressed against the surface of a subject's body, can be removable and replaced with another tip that contains a sensor that provides additional functionality. For example, a standard device (1) having just pressure and displacement sensors (5,6) can have a probe (3) with a removeable tip (23), which would allow another tip (23) to be added that contains a thermoelectric element and/or temperature sensor and/or a sensor to measure epidermal skin response. Means for removing and attaching such a tip would be well known in the art, and can include compression fitting the tip to the rest of the probe or screw fitting the tip to the probe.

In the case where the various sensors are electronic, they can be connected to a microprocessor (24), which can be configured to receive data from the sensors. In one embodiment, the microprocessor (24) transmits the data to an output, which can be, but not limited to, a display (25) positioned on the exterior of the housing (2), a transmitter, and/or a wired connection to a computer or tablet. The transmitter can be in the form of a WiFi or Bluetooth^{®} transmitter that transmits the data to a remote terminal, such as a computer, tablet or phone. In another embodiment, the sensors are directly connected to the microprocessor via a wire.

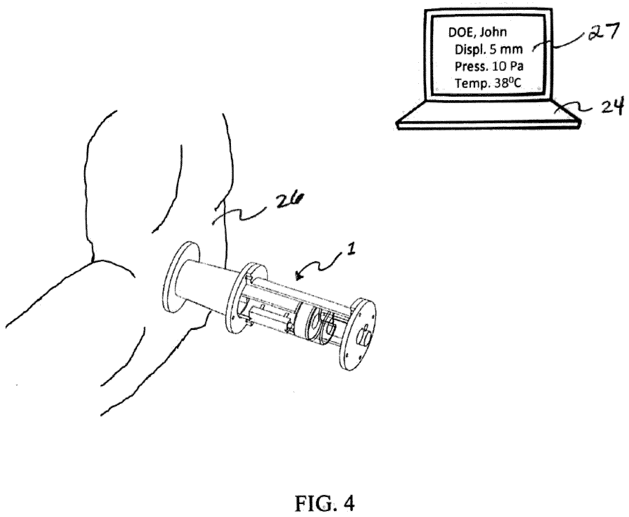

In operation, as shown in FIG. 4, the device (1) is positioned against a target area of the subject's body (26). If the device (1) is provided with plate (8), as described above, the target area is centered within the plate (8) so that the probe (3) makes direct contact with the target area. The operator of the device (1) then engages the activator (4) by pressing the activator (4) towards the housing (2) thus causing the probe (3) to be moved from the first position, where the probe (3) is entirely or mostly encompassed within the housing (2), and the second position, where the probe (3) extends beyond the subject engagement end (7) of the housing (2). The operator continues to press down on the activator (4) and asks the subject to describe, typically on a scale of 1 to 10, the amount of pain or discomfort they are feeling. Alternatively, the operator can also ask the subject to let them know when they feel a certain level of pain or discomfort. The data provided by the subject with respect to the sensitivity that they feel can be inputted into a microprocessor (24) along with the measurements and data obtained by the pressure and displacement sensors (5, 6) of the device (1). This allows a correlation to be made between depth and pressure applied by the probe (3) to the amount of sensitivity, in terms of pain and discomfort, perceived by the subject. Over time, the readings can reliably show whether the subject is becoming truly less sensitive to pain or discomfort. In addition, the sensitivity measurements can be standardized from one patient to the next by using select areas on the body, such as trigger points, which are well known in the art, to identify a subject's overall sensitivity to pain.

In another embodiment, a sensor may be placed in the probe to measure a subject's galvanic skin response (GSR), originating from the autonomic activation of sweat glands, which is an indication of emotional arousal of the subject. The GSR measurements can be sent to the microprocessor and recorded along with the subject's other measurements.

As described above, the probe (3) can also contain a temperature sensor to measure the temperature of the subject's skin at the target area. This temperature data can also be fed into the microprocessor (24) along with the pressure and displacement data, and used to further supplement the subject's sensitivity profile. Similarly, a thermoelectric element can be used to obtain data with respect to the subject's sensitivity to heat and/or cold. This data can also be transmitted to the microprocessor (24) to be included in the subject's sensitivity profile.

The microprocessor that receives the pressure, displacement and, optionally, the GSR and temperature data can be either part of a display, such as a tablet or phone, or can be connected to a display show that the set of values (27) received from the hand held device (1) can be observed. The location of the target area on the subject's body can also be inputted into the microprocessor or can be recalled from computer readable medium present in the device or from separate device, such as a server, smart device or web application. The set of values (27) can be saved to a computer readable medium under the subject's profile. This information can be recalled to determine whether the subject's sensitivity to pain or discomfort is improving over time.

The present invention has been described with regard to one or more embodiments, However, it will be apparent to persons skilled in the art that a number of variations and modifications can be made without departing from the scope of the invention.

## Claims

1. A hand-held device for taking measurements related to sensitivity in a subject, said hand-held device comprising:
a housing having a subject engagement end for positioning the device against a target area of the subject's body;
a probe encompassed by the housing and moveable from a first position where the probe is contained within the housing to a second position where one end of the probe extends beyond the subject engagement end of the housing;
an activator positioned on an exterior surface of the housing and connected to the probe for moving the probe between the first and second positions;
a displacement sensor connected to the probe for measuring the distance the probe travels between the first and second positions; and
a pressure sensor connected to the probe for measuring the force applied to move the probe between the first and second positions.

2. The hand-held device of claim 1, further comprising a microprocessor connected to the displacement and pressure sensors for receiving measurements from the displacement and pressure sensors; and an output for relaying information from the microprocessor to a user.

3. The hand-held device of claim 1 or 2, wherein the displacement sensor comprises a rod having distance intervals marked thereon to provide a visual indication of the distance the probe travels between the first and second positions.

4. The hand-held device of any one of claims 1 to 3, further comprising a temperature sensor connected to the one end of the probe for measuring the temperature of the target area of the subject's body.

5. The hand-held device of claim 4, wherein the temperature sensor is connected to the microprocessor.

6. The hand-held device of claim 2, wherein the output is a transmitter or a display positioned on the exterior of the housing.

7. The hand-held device of claim 6, wherein the transmitter is a WiFi or Bluetooth^{®}.

8. The hand-held device of claim 2, wherein the output is a wire connected to computer or tablet, and optionally the wire is removably connected to the output and/or computer or tablet.

9. The hand-held device of any one of claims 1 to 8, wherein a portion of the exterior surface of the housing forms a grip for the user's hand.

10. The hand-held device of any one of claims 1 to 9, wherein the activator is positioned on or near the end of the housing opposite the subject engagement end.

11. The hand-held device of any one of claims 1 to 10, wherein the subject engagement end of the housing comprises a plate to surround the target area of the subject's body.

12. The hand-held device of any one of claims 1 to 11, wherein the displacement sensor and pressure sensor are piezoelectric sensors.

13. A system for measuring and tracking sensitivity in a subject, said system comprising:
the hand-held device of any one of claims 1 to 12;
a microprocessor connected to the hand-held device;
an input device connected to the microprocessor for inputting data related to the subject's sensitivity to the probe being pressed against the target area; and
a display connected to the microprocessor for displaying a set of values.

14. The system of claim 13, wherein the microprocessor is wireless connected to the hand-held device or contained within a computer, tablet or cellphone.

15. The system of claim 13 or 14, wherein the set of values relates to one or more of the force measured by the pressure sensor, the distance travelled by the probe, the temperature of the target area and the data related to the subject's sensitivity to the probe being pressed against the target area.

16. The system of any one of claims 13 to 15, wherein the microprocessor comprises computer-readable medium for storing the set of values.

17. Use of the device of any one of claims 1 to 12, or the system of any one of claims 13 to 16, for taking measurements related to sensitivity in a subject.
